# EUROPÄISCHE PATENTSCHRIFT

(11) **EP 2 158 881 B1**
(45) Veröffentlichungstag und Bekanntmachung des Hinweises auf die Patenterteilung: **24.04.2013**
(21) Anmeldenummer: 09009424.4
(22) Anmeldetag: 21.07.2009
(51) Int. Cl.: A61F 5/11

(54) **Vorrichtung zur Nagelkorrektur**
Device for correcting nails
Dispositif destiné à la correction d'ongle

(30) Priorität: 26.08.2008 DE 102008039762
(43) Veröffentlichungstag der Anmeldung: 03.03.2010
(73) Patentinhaber: 3TO GmbH, 82041 Deisenhofen (DE)
(72) Erfinder: Sutor, Norbert, 82041 Deisenhofen (DE); Sutor, Franziska, 80333 München (DE); Sutor, Johannes, 80339 München (DE)
(74) Vertreter: Müller, Bernhard

(56) Entgegenhaltungen:
- EP-A- 1 849 442
- EP-A1- 2 221 029
- WO-A-2007/102156
- US-A- 2 920 621
- GREPPMAYR G: "Die Wege zur Orthonyxie", FUSS, VERLAG NEUER MERKUR GMBH, vol. 9, 1 March 1968 (1968-03-01), pages 5-6, XP007920740, ISSN: 1430-8886

## Beschreibung

Die Erfindung betrifft eine Vorrichtung zur Nagelkorrektur gemäß Oberbegriff des Hauptanspruchs.

Zur Behandlung von eingewachsenen oder eingerollten Nägeln, insbesondere Großzehennägeln, wurden als Alternative zur schmerzhaften und mit langen Genesungszeiten verbundenen Operation (Emmert-Plastik) verschiedene Nagelkorrekturspangen entwickelt. Diese so genannten Orthonyxiespangen reduzieren die Krümmung der Nagelplatte und entlasten dadurch den oftmals entzündeten Nagelfalz. Die Schmerzen werden deutlich gemindert und eine Abheilung ermöglicht.

Die Wirkung dieser Nagelkorrekturspangen beruht auf Rückstellkräften, außermittigen Zugkräften oder auf Kombinationen dieser beiden Mechanismen.

Folgende Vorrichtungen zur Nagelkorrektur sind bisher bekannt und werden zur Behandlung eingesetzt. Sie weisen jedoch entweder bezüglich ihrer Wirksamkeit oder ihrer Anwendung einige Nachteile auf:
**Drahtspange aus einem Teil (Fraserspange)**

Die so genannte Fraserspange ist eine einteilige Drahtspange, versehen mit Häkchen, welche unter den Nagelrand greifen und diesen mittels Rückstellkraft anheben. Diese Spange muss vom Therapeuten für jeden Patienten individuell angefertigt werden. Dies erfolgt unter erheblichem Zeitaufwand an einem zuvor anhand eines Abdruckes herzustellenden Modell. Die Vorrichtung wird von vorne auf den Nagel aufgeschoben, was eine exakte Anpassung auf die Nagelbreite erfordert und zudem ein erhebliches Verletzungsrisiko im engen oder gar entzündeten Nagelfalz darstellt. Die Wirkungskraft kann bei dieser Vorrichtung nur äußerst ungenau und nur durch sehr erfahrene Anwender eingestellt werden.
**Drahtspange aus 3 Teilen (2 Schenkel + 1 Schlaufe;** DE42 07 797A1**)**

Eine Weiterentwicklung der Fraserspange stellt die dreiteilige Drahtspange dar, welche aus zwei mit Häkchen versehenen Schenkeln und einer in der Mitte anzubringenden Drahtschlaufe besteht. Der Therapeut versieht die teilweise vorgefertigten Spangenschenkel mit Häkchen (oder passt diese im Falle einer industriellen Vorfertigung derselben an die Nageldicke an) und formt die Spange entsprechend der Nagelkrümmung. Die Schenkel werden einzeln unter den Nagelrand eingehängt und mit der vorgefertigten Schlaufe verbunden, wodurch die Spangenbreite an die Nagelbreite angepasst und die Spangenkraft stufenlos in Absprache mit dem Patienten eingestellt werden kann.

Die Anwendung dieser Spangen erfordert großes Geschick des Therapeuten und macht eine spezielle Schulung unumgänglich. Zudem muss die Spange stets für die jeweilige Nagelbreite ausgesucht oder angepasst werden.

Auf Grund der geringen Abmessungen ist zudem das Anbringen der Mittelschlaufe relativ aufwändig.
**Klebespangen aus Kunststoff oder Metall (**DE37 08 811A1**; BS-Spange)**

Auf die Rückstellkraft von Metall- oder Kunststoffelementen basierende Klebespangen werden im vorgespannten Zustand auf die Nagelplatte aufgeklebt. Dafür muss die Spange an den Nagel angedrückt werden, was bei ohnehin schmerzhaft eingewachsenen Nägeln mit erheblichen Schmerzen für den Patienten verbunden sein kann. Bei zu geringem Anpressdruck hingegen ist die Spange nicht ausreichend fixiert und kann sich wieder lösen.

Je nach System wird die Intensität der Spangenkraft durch die Vorauswahl verschiedener Querschnitte oder durch Verringerung der Spangendicke im aufgeklebten Zustand variiert. Der Kraftangriff erfolgt nicht in den äußersten Randbereichen, da diese meist nicht sichtbar im Nagelfalz verborgen liegen.

### Nagelkorrekturspange bestehend aus 2 Ankerplättchen mit einem losen Zugelement

Eine Vorrichtung bestehend aus zwei, auf den Nagel aufgeklebten Ankern, welche durch ein loses Zugelement miteinander verbunden werden ist aus der DE 32 33 419 A1 bekannt. Hier können die Elemente zur Krafteinleitung im ungespannten Zustand auf die Nagelplatte geklebt werden. Die Einstellung der Spangenkraft erfolgt über ein elastisches Zugelement, wie zum Beispiel einen Gummiring, wobei die Intensität der Spangenkraft vom Abstand der Befestigungselemente und der Größe und Stärke des Zugelements abhängt und daher nicht genau und nicht stufenlos einstellbar ist.

Auf Grund der relativ hohen Ausführung der Ankerplatten kann durch enge Schuhe Druck auf den Nagel übertragen werden, zudem kann das nur lose befestigte Zugelement verloren werden.

**Nagelkorrekturspange mit aufklebbaren Haftelementen und eingebettetem Zugelement (podofix-Spange)**

Eine aufklebbare Nagelspange mit der Möglichkeit der spannungsfreien Anbringung und anschließender stufenloser Aktivierung ist als so genannte podofix-Spange bekannt. Dem Vorteil der stufenlosen Regulierung steht der Nachteil der nur im sichtbaren Bereich des Nagels erzeugbaren Wirkung gegenüber, da die Spange nicht unter den Nagelrand greift.

Aus G. Greppmayr, "Die Wege zur Orthonyxie", der fuss, Verlag Neuer Merkur GmbH, Bd. 9 (1968), S. 5-6, ist eine Nagelkorrekturvorrichtung bekannt, die einen unter den Nagelrand einzuhängenden Haken C aus federhartem Draht umfasst, der mit einer auf die Nagelplatte aufzuklebenden Drahtgaze verbunden werden kann. Manipulationshilfen sind bei dieser Vorrichtung nicht vorgesehen.

US-2 920 621 betrifft eine Vorrichtung zur Nagelkorrektur, die aus einem hakenförmigen Element in Form eines Metallbleches zum Einführen unter den Rand eines Zehennagels besteht, wobei dieses Band mittels eines Klebebands auf den Nagel gedrückt wird, und dadurch eine Kraft auf den Haken und damit auch auf den Nagelrand ausgeübt wird. Das hakenförmige Element selbst ist vorzugsweise lose und nicht verklebt, wie sich aus Spalte 2, Zeilen 55-63, ergibt.

WO-2007/102156 betrifft eine Vorrichtung zur Nagelkorrektur, bei der ein so genanntes "releasing element" 14 mit einem auf die Oberfläche des Nagels aufklebbaren "supporting element" 12 verbunden ist. Diese beiden Elemente sind mittels eines elastischen Elements 18 miteinander verbunden, das das "releasing element" nach oben zieht und somit den Rand des Nagels vom eingewachsenen Bereich tendenziell nach oben hebt. Eine derartige Spange kann nicht unter dem Nagelrand wirken. Vielmehr wirkt sie nur im sichtbaren Bereich.

EP 1 849 442 A1 betrifft eine Vorrichtung zur Nagelkorrektur, die mindestens zwei hakenförmige Krafteinleitungselemente, die unter den Rand eines Nagels einhängbar sind, und mindestens ein Verbindungselement aufweist, wobei das oder die verbindungselemente flexibel sind und fest an einem oder zwei Krafteinleitungslementen angebracht sind. Dabei soll auf die Krafteinleitungselemente eine stufenlos einstellbare Zugkraft einwirken, die durch außermittigen Kraftangriff ein Biegemoment auf die Nagelplatte überträgt. Von der Ausübung eines Biegemoments durch Einwirken einer Rückstellkraft ist in dieser Druckschrift nicht die Rede.

Die nicht vorveröffentlichte Druckschrift EP 2 221 029 A1 betrifft eine Vorrichtung zur Nagelkorrektur, die ein bandförmiges, zu einem Haken geformtes Krafteinleitungselement und ein auf den Nagel aufklebbares Haftelement umfasst. Die Verwendung eines federharten Drahts als Krafteinleitungselement wird in dieser Druckschrift nicht erwähnt.

Aufgabe der vorliegenden Erfindung ist es, eine Vorrichtung zur Nagelkorrektur zu schaffen, welche einfach und schnell anwendbar ist und gleichzeitig die Kraft auf den Nagel unter dem Nagelrand, dem für die Therapie bedeutendsten Bereich, einbringt.

Im Laufe von Entwicklungsarbeiten im Rahmen der vorliegenden Erfindung wurde festgestellt, dass es von besonderem Vorteil ist, den unter den Nagelrand greifenden Haken einer Drahtspange mit einem aufklebbaren Haftelement zu verbinden.

Der Gegenstand der Erfindung ist im Anspruch 1 definiert.

Fortentwicklungen und besondere Ausführungsformen der Erfindung ergeben sich aus den Unteransprüchen.

Nachstehend wird die Erfindung beispielhaft anhand einiger Zeichnungen näher erläutert:
Dabei zeigen:
   Fig. 1 eine einteilige Ausführung der erfindungsgemäßen Vorrichtung zur Nagelkorrektur im montierten Zustand;
   Fig. 2 eine zweigeteilte Ausführung der erfindungsgemäßen Vorrichtung zur Nagelkorrektur im montierten Zustand;
Figg. 3 und 4 Ausführungsbeispiele der Haftelemente der in Figur 2 dargestellten Ausführung;
Fig. 5 ein Haftelement mit einem damit verbundenen Griff als Manipulationshilfe;
Fig. 6 eine weitere Ausführungsform einer einteiligen Vorrichtung zur Nagelkorrektur mit einer integrierten elastischen Komponente.

Nachstehend wird auf die in den Figuren dargestellten Ausführungsformen näher eingegangen.

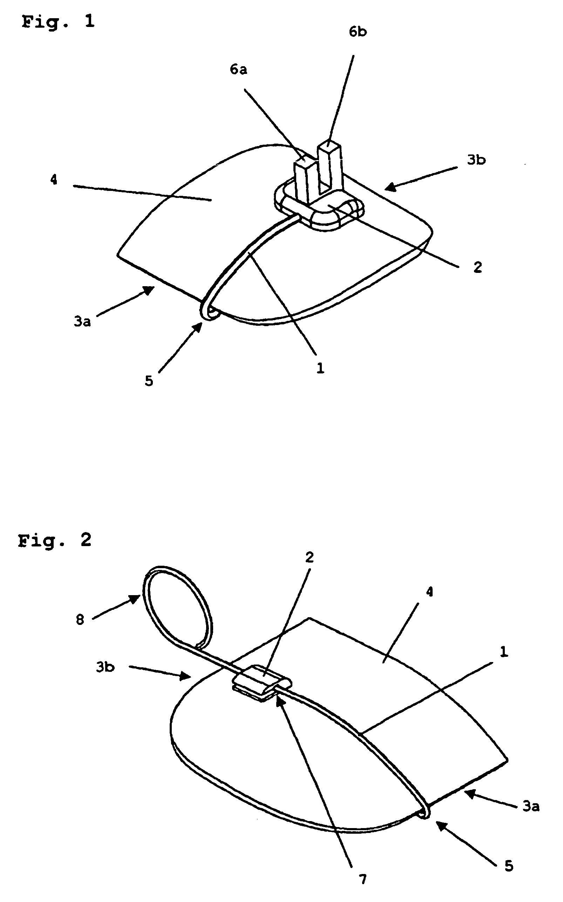

Figur 1 zeigt die erfindungsgemäße Vorrichtung im auf den Nagel montierten Zustand. Zur Behandlung des eingewachsenen Nagels 4 einer menschlichen Zehe sollen die Nagelränder 3a, 3b minimal angehoben werden, um den schmerzenden Nagelfalz zu entlasten. Hierfür greift ein, am bevorzugt aus federhartem Draht bestehenden Krafteinleitungselement 1 befindliches Häkchen 5 unter einen Nagelrand 3a. Das Haftelement 2 ist in der dargestellten Ausführung unlösbar mit dem Krafteinleitungselement 1 verbunden und besteht in einer bevorzugten Ausführung aus einem elastischen Kunststoff. Zur festen Verbindung kann das Haftelement 2 direkt an das Verbindungselement 1 angespritzt sein. Das Haftelement 2 ist mittels eines schnell aushärtenden Klebstoffes auf der Oberfläche des Nagels 4 fixiert.

Auf Grund der Rückstellkraft des aus federhartem Material bestehenden Krafteinleitungselementes 1 im Bereich zwischen dem Häkchen 5 und dem Haftelement 2 wirkt auf den Nagel 4 ein Biegemoment, die Nagelränder 3a, 3b werden angehoben. Die Intensität der Rückstellkraft kann vom Anwender der erfindungsgemäßen Vorrichtung zur Nagelkorrektur durch mehr oder minder starke Krümmung des Krafteinleitungselementes 1 und die Auswahl unterschiedlicher Materialquerschnitte desselben beeinflusst werden.

Zur einfacheren Handhabung sind auf der Oberseite des Haftelements 2 zwei Zapfen 6a, 6b angeordnet. Diese Zapfen 6a, 6b werden nach dem Aufkleben des Haftelements 2 auf den Zehennagel abgeschnitten, so dass das Haftelement 2 eine ebene Oberfläche aufweist.

Figur 2 zeigt eine zweigeteilte Ausführung der erfindungsgemäßen Vorrichtung zur Nagelkorrektur. Bei dieser Ausgestaltung ergibt sich der Vorteil, dass das Haftelement 2 zunächst spannungsfrei auf den Nagel 4 aufgeklebt werden kann und das Krafteinleitungselement 1 anschließend durch formschlüssige Verbindung am Haftelement 2 befestigt werden kann. Das Haftelement 2, welches bevorzugt aus einem zähelastischen Kunststoff gefertigt ist, weist eine Hinterschneidung 7 auf, welche zur Aufnahme des Krafteinleitungselementes 1 dient. Das Kraftelement 1 besteht aus federhartem Draht und ist zum Einhängen unter den Nagelrand 3a je nach Ausführung an einem Ende bereits mit einem teilweise oder vollständig vorgeformten Häkchen 5 versehen, oder zunächst gerade ausgeführt, so dass das Häkchen an beliebiger Stelle vom Anwender angebogen werden kann. Das dem Häkchen 5 gegenüberliegende Ende des Krafteinleitungselementes 1 kann zum erleichterten Einführen in die Hinterschneidung 7 des Haftelementes 2 beispielsweise mit einer Öse 8 versehen sein, welche nach der Montage abgetrennt wird. Die endgültige Fixierung des Krafteinleitungselementes 1 im Haftelement 2 kann durch eine Versiegelung mit einem Klebstoff oder einer aushärtenden Kunststoffmasse vorgenommen werden.

### Ausführungsformen der Haftelemente

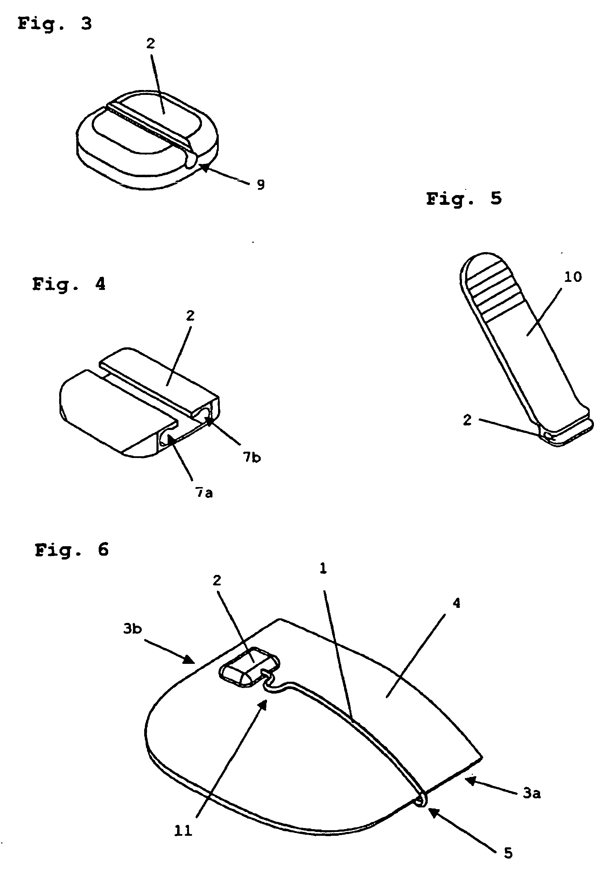

In Figur 3 ist eine Ausführung des Haftelementes dargestellt, bei welchem das aus Draht bestehende Krafteinleitungselement in die leicht hinterschnittene Kammer 9 des Haftelementes 2 hineingedrückt und dort durch die Spannkraft des elastischen Kunststoffes festgehalten wird. Zusätzlich kann bei Bedarf eine endgültige Fixierung durch Klebstoff erfolgen.

Figur 4 zeigt ein Haftelement 2 mit zwei Hinterschneidungen 7a, 7b, welche zur Aufnahme von zwei Krafteinleitungselementen dienen. So kann mit einem mittig aufgeklebten Haftelement je ein Krafteinleitungselement zu jeder Nagelseite angebracht werden.

### Manipulationshilfen

Zur Vereinfachung der Handhabung beim Anbringen der Vorrichtung zur Nagelkorrektur wird diese mit Manipulationshilfen versehen. Diese können entweder zur Handhabung mit Werkzeugen oder zur werkzeuglosen Handhabung dienen.

Wie bereits erwähnt, dienen, bei der in Figur 1 dargestellten Ausführung zwei auf der Oberseite des Haftelementes 1 angebrachte Zapfen 6a, 6b als Greifhilfe für eine Zange, um das Aufsetzen und Ankleben des Haftelementes 1 zu erleichtern. Die Zapfen 6a, 6b werden nach der Applikation der Vorrichtung zur Nagelkorrektur mittels Seitenschneider oder Skalpell abgetrennt.

Zur werkzeuglosen Montage kann die in Figur 2 dargestellte an den Draht des Krafteinleitungselementes 1 angebogene Öse 8 verwendet werden. Diese Öse kann sowohl in der zweigeteilten Ausführung der Vorrichtung zur Nagelkorrektur zum Einhängen des drahtförmigen Krafteinleitungselementes in das Haftelement dienen, als auch das Aufkleben des Haftelementes der einteiligen Ausführung mit den Fingern ermöglichen. Die Manipulationshilfe wird nach der Montage von der Vorrichtung abgetrennt.

In Figur 5 ist eine Ausführung mit einem mit dem Haftelement 2 verbundenen Griff 10 dargestellt. Dieser Griff 10 dient zum Greifen mit den Fingern, um das Aufkleben des Haftelementes 2 zu vereinfachen. Der Griff 10 wird nach dem Anbringen der Vorrichtung zur Nagelkorrektur mit einem Seitenschneider vom Haftelement 2 abgetrennt.

### Elastische Komponenten

Figur 6 zeigt beispielhaft eine Möglichkeit der Einbindung von zusätzlichen elastischen Komponenten in die Vorrichtung zur Nagelkorrektur, um die beim Gehen des Patienten entstehenden Verformungen des Nagels 4 auszugleichen. Diese können beispielsweise als Omega-förmige Biegung 11 im Draht des Krafteinleitungselementes 1 ausgeführt sein.

### Vorteile der erfindungsgemäßen Vorrichtung zur Nagelkorrektur

Gegenüber den bisher bekannten, herkömmlichen Verfahren und Vorrichtungen weist die erfindungsgemäße Vorrichtung zur Nagelkorrektur einige Vorteile auf.

Durch ihre schnelle und einfache Anwendung wird diese Nagelkorrekturspange der Anforderung der Wirtschaftlichkeit in der ärztlichen, podologischen und kosmetischen Praxis besonders gerecht.

Durch die Verbindung eines mit einem Häkchen versehenen Krafteinleitungselementes mit einem aufklebbaren Haftelement ist eine gezielte Behandlung auch stark verformter Nägel möglich. Somit kann auch eine einzelne Seite des Nagels mit einer definierten Kraft beaufschlagt werden.

Zudem kann je nach vorliegendem Befund der Nagelrand von einem Häkchen umfasst werden oder die Kraft durch das aufgeklebte Haftelement in den Nagelrand eingeleitet werden. Vorteilhaft ist dies bei häufig einseitig eingewachsenen Nägeln, bei welchen der Einsatz von Drahthäkchen im entzündeten Bereich stark umstritten ist.

In ihrer zweigeteilten Ausführung ist die Vorrichtung zur Nagelkorrektur besonders einfach zu montieren, da hier das Haftelement im Gegensatz zu den meisten am Markt befindlichen Klebespangen ohne Spannung und daher deutlich sicherer aufgeklebt werden kann.

Auf Grund ihrer sehr flachen Bauweise wird diese Vorrichtung zur Nagelkorrektur auch in engen Schuhen nicht als störend empfunden.

## Patentansprüche

1. Vorrichtung zur Nagelkorrektur, bestehend aus mindestens einem aus federhartem Stahl bestehenden hakenförmigen Krafteinleitungselement (1), welches unter den Rand (3a) eines Zehennagels (4) einhängbar ist, wobei das hakenförmige Krafteinleitungselement (1) mit einem auf die Oberfläche des Nagels (4) aufklebbaren Haftelement (2) verbunden ist und dadurch mittels Rückstellkräften ein Biegemoment auf die Nagelplatte übertragen kann,
**dadurch gekennzeichnet, dass** an der Vorrichtung Elemente zur Manipulationshilfe angebracht sind.

2. Vorrichtung nach Anspruch 1, **dadurch gekennzeichnet, dass** das Krafteinleitungselement (1) fest mit dem aufklebbaren Haftelement (2) verbunden ist.

3. Vorrichtung nach Anspruch 1, **dadurch gekennzeichnet, dass** das Krafteinleitungselement (1) unabhängig vom Haftelement (2) anbringbar und an diesem durch Kraft- und / oder Formschluss fixierbar ist.

4. Vorrichtung nach einem der vorstehenden Ansprüche, **dadurch gekennzeichnet, dass** der Haken des Krafteinleitungselementes (1) vom Anwender geformt wird.

5. Vorrichtung nach einem der Ansprüche 1 bis 3, **dadurch gekennzeichnet, dass** der Haken des Krafteinleitungselementes (1) bereits ganz oder teilweise vorgeformt werden kann.

6. Vorrichtung nach einem der vorstehenden Ansprüche, **dadurch gekennzeichnet, dass** das Krafteinleitungselement (1) elastische Komponenten aufweist.

7. Vorrichtung nach einem der vorstehenden Ansprüche, **dadurch gekennzeichnet, dass** das Haftelement aus einem elastischen Kunststoff besteht.

8. Vorrichtung nach Anspruch 1, **dadurch gekennzeichnet, dass** die Manipulationshilfe zwei auf der Oberfläche des Haftelements (2) ausgebildete Zapfen (6a, 6b) umfasst.

9. Vorrichtung nach Anspruch 1, **dadurch gekennzeichnet, dass** die Manipulationshilfe einen mit dem Haftelement (2) verbundenen Griff (10) umfasst.

10. Vorrichtung nach Anspruch 1, **dadurch gekennzeichnet, dass** die Manipulationshilfe eine mit dem Krafteinleitungselement (1) verbundene Öse (8) umfasst.

## Claims

1. Device for nail correction consisting of at least one hook-like force introduction member (1) which consists of spring-hard steel and is adapted to be engaged beneath the edge (3a) of a toe nail (4), wherein the hook-like force introduction member (1) is connected to a bonding member (2) adherable to the surface of the nail (4) and thereby by means of restoring forces a bending moment can be transmitted to the nail body, **characterized in that** members for handling aid are provided on the device.

2. Device according to claim 1, **characterized in that** the force introduction member (1) is fixedly connected to the adherable bonding member (2).

3. Device according to claim 1, **characterized in that** the force introduction member (1) can be attached independently of the adherable bonding member (2) and is adapted to be fixed to the latter by nonpositive and/or positive or form-locking.

4. Device according to any one of the preceding claims, **characterized in that** the hook of the force introduction member (1) is formed by the user.

5. Device according to any one of claims 1 to 3, **characterized in that** the hook of the force introduction member (1) can already be completely or partially preformed.

6. Device according to any one of the preceding claims, **characterized in that** the force introduction member (1) comprises elastic components.

7. Device according to any one of the preceding claims, **characterized in that** the bonding member consists of an elastic plastic.

8. Device according to claim 1, **characterized in that** the handling aid includes two studs (6a, 6b) formed on the surface of the bonding member (2).

9. Device according to claim 1, **characterized in that** the handling aid includes a grip (10) connected to the bonding member (2).

10. Device according to claim 1, **characterized in that** the handling aid includes an eye (8) connected to the force introduction member (1).

## Revendications

1. Dispositif destiné à la correction d'ongle, constitué d'au moins un élément d'introduction de force (1) en forme de crochet, lequel est constitué d' un fil d'acier écroui et peut être accroché sous le bord (3a) d'un ongle d'orteil (4), sachant que l'élément d'introduction de force (1) en forme de crochet est relié à un élément adhésif (2) pouvant être collé sur la surface de l'ongle (4) et que de ce fait un moment de flexion peut être transmis sur le corps d'ongle au moyen de forces de rappel, **caractérisé en ce que** des éléments servant d'aide à la manipulation sont présents sur le dispositif.

2. Dispositif selon la revendication 1, **caractérisé en ce que** l'élément d'introduction de force (1) est relié de manière fixe à l'élément adhésif (2) pouvant être collé.

3. Dispositif selon la revendication 1, **caractérisé en ce que** l'élément d'introduction de force (1) peut être appliqué indépendamment de l'élément adhésif (2) et peut être fixé à ce dernier par complémentarité de force et/ou de forme.

4. Dispositif selon l'une quelconque des revendications précédentes, **caractérisé en ce que** le crochet de l'élément d'introduction de force (1) est formé par l'utilisateur.

5. Dispositif selon l'une quelconque des revendications 1 à 3, **caractérisé en ce que** le crochet de l'élément d'introduction de force (1) peut être déjà complètement ou partiellement préformé.

6. Dispositif selon l'une quelconque des revendications précédentes, **caractérisé en ce que** l'élément d'introduction de force (1) présente des composants élastiques.

7. Dispositif selon l'une quelconque des revendications précédentes, **caractérisé en ce que** l'élément adhésif est constitué d'un plastique élastique.

8. Dispositif selon la revendication 1, **caractérisé en ce que** l'élément d'aide à la manipulation comporte deux tourillons (6a, 6b) réalisés sur la surface de l'élément adhésif (2).

9. Dispositif selon la revendication 1, **caractérisé en ce que** l'élément d'aide à la manipulation comporte un moyen de préhension (10) relié à l'élément adhésif (2).

10. Dispositif selon la revendication 1, **caractérisé en ce que** l'élément d'aide à la manipulation comporte un oeillet (8) relié à l'élément d'introduction de force (1).
